# EUROPÄISCHE PATENTSCHRIFT

(11) **EP 1 203 043 B1**
(45) Veröffentlichungstag und Bekanntmachung des Hinweises auf die Patenterteilung: **08.10.2003**
(21) Anmeldenummer: 00945886.0
(22) Anmeldetag: 06.07.2000
(51) Int. Cl.: C08G 64/06, C08G 64/24, C08G 64/26

(54) **OLIGOMERE BISCHLORKOHLENSÄUREESTER AUS AUSGEWÄHLTEN CYCLOALIPHATISCHEN BISPHENOLEN UND ANDEREN BISPHENOLEN**
OLIGOMERIC BISCHLOROCARBONIC ACID ESTERS OBTAINED FROM SELECTED CYCLOALIPHATIC BISPHENOLS AND OTHER BISPHENOLS
ESTERS OLIGOMERES D'ACIDE BISCHLOROCARBONIQUE OBTENUS A PARTIR DE BISPHENOLS CYCLOALIPHATIQUES SELECTIONNES ET D'AUTRES BISPHENOLS

(30) Priorität: 19.07.1999 DE 19933070; 22.12.1999 DE 19962016
(43) Veröffentlichungstag der Anmeldung: 08.05.2002
(73) Patentinhaber: Bayer Aktiengesellschaft, 51368 Leverkusen (DE)
(72) Erfinder: EBERT, Wolfgang, D-47800 Krefeld (DE); KÖHLER, Burkhard, D-51373 Leverkusen (DE); CHEN, Yun, A18-DF, Celebrity Garden Shanghai, 201612 Shanghai (CN)
(86) Internationale Anmeldenummer: EP0006380
(87) Internationale Veröffentlichungsnummer: WO01005869

(56) Entgegenhaltungen:
- EP-A- 0 362 792
- EP-A- 0 414 083
- EP-A- 0 691 361
- EP-A- 0 827 948
- DE-B- 1 301 908
- US-A- 4 920 200

## Beschreibung

Die Erfindung betrifft oligomere Bischlorkohlensäureester, die in einer Zweiphasenreaktion aus ausgewählten cycloaliphatischen Bisphenolen und anderen Bisphenolen durch Einleiten von Phosgen zugänglich sind.

Bischlorkohlensäureester eignen sich als Edukte zur Herstellung von Polycarbonatcyclen (als Vorstufe für hochmolekulare Polycarbonate gemäß DE 19 636 539) und von linearen Polycarbonaten, z.B. aus Lösungspolymerisation.

Das Bisphenol 1,1-Bishydroxyphenyl-3,3,5-trimethylcyclohexan (TMC-Bisphenol) ergibt Polycarbonate mit hoher Glastemperatur. Oft sind wegen der besseren Verarbeitbarkeit Polycarbonate mit einer niedrigeren Glastemperatur erwünscht.

In Frage kommen z.B. Copolymere mit anderen Bisphenolen.

Prinzipiell ist es möglich, eine oligomere Bischlorkohlensäureestervorstufe des TMC-Bisphenols (Vorstufe 1; V1) mit einer anderen oligomeren Bischlorkohlensäureestervorstufe (Vorstufe 2; V2) vor der Reaktion zu mischen. Jedoch entsteht dann bei der Folgereaktion keine statistische Sequenz der monomeren Bisphenole (V1) und (V2), sondern Blöcke der Vorstufe 1 und der Vorstufe 2. Daher ist es wünschenswert, oligomere Bischlorkohlensäureestervorstufen herzustellen, die selbst statistisch aus TMC-Bisphenol und anderen Bisphenolen aufgebaut sind.

Gegenstand der Erfindung sind daher Gemische aus Bischlorkohlensäureestern der Formeln (I), (II) und (III) 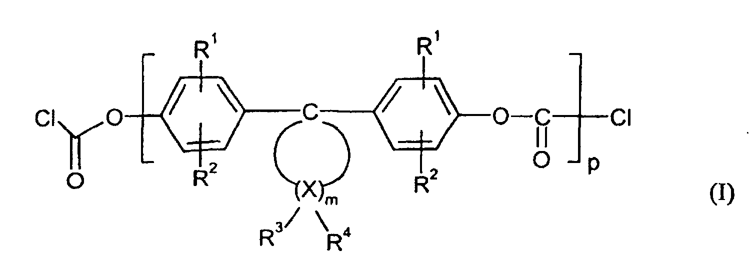 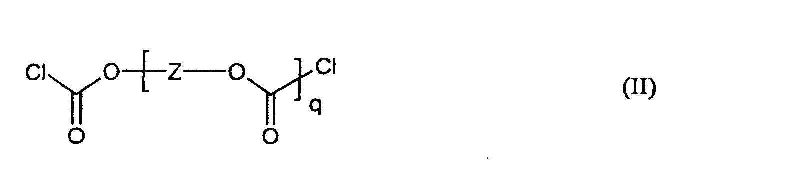 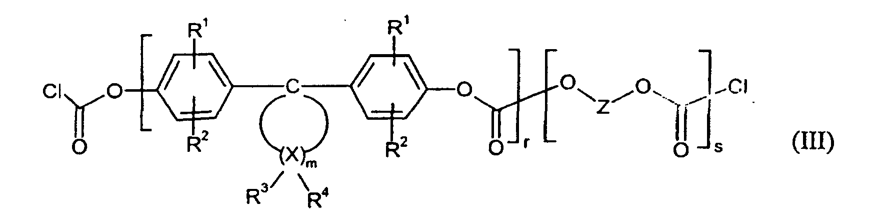 worin
- R¹ und R²: unabhängig voneinander Wasserstoff, Halogen, bevorzugt Chlor oder Brom, C₁-C₈-Alkyl, C₅-C₆-Cycloalkyl, C₆-C₁₀-Aryl, bevorzugt Phenyl, und C₇-C₁₂-Aralkyl, bevorzugt Phenyl-C₁-C₄-Alkyl, insbesondere Benzyl,
- m: eine ganze Zahl von 4 bis 7, bevorzugt 4 oder 5,
- R³ und R⁴: unabhängig voneinander Wasserstoff oder C₁-C₆-Alkyl und
- X: Kohlenstoff bedeuten,
mit der Maßgabe, dass an mindestens einem Atom X pro Wiederholungseinheit in (I) oder (III), R³ und R⁴ gleichzeitig Alkyl bedeuten,
Z ein aromatischer Rest mit 6 bis 30 C-Atomen ist, der einen oder mehrere aromatische Kerne enthalten kann, substituiert sein kann und aliphatische Reste oder cycloaliphatische Reste, die unterschiedlich zu den cycloaliphatischen Resten in (I) sind, oder Heteroatome als Brückenglieder enthalten kann,
p, q, r und s für eine natürliche Zahl von 1 bis 15 stehen und das molare Verhältnis der cycloaliphatischen Bisphenoleinheiten zu den aliphatischen Bisphenoleinheiten 1:10 bis 10:1 beträgt.

Bevorzugt ist Z 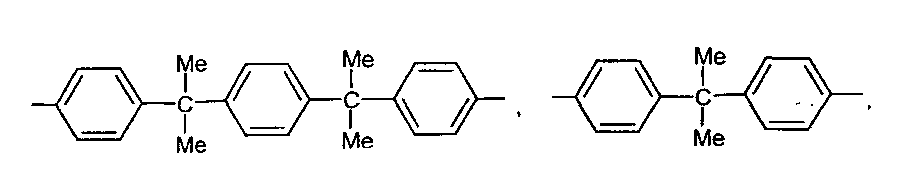 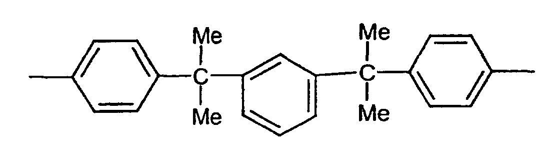 mit Me = Methylrest

Die Herstellung der Bischlorkohlensäureestergemische erfolgt bevorzugt durch Einleiten von Phosgen in eine Mischung von Bisphenolen der Formeln (IV) und (V) im molaren Verhältnis von 1:10 bis 10:1.

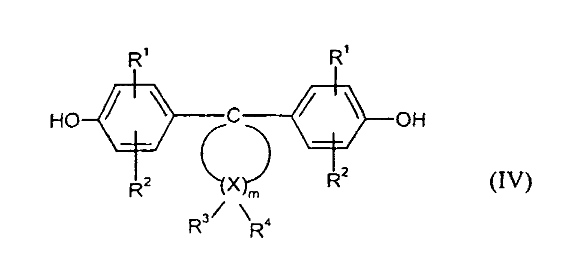

HO-Z-OH (V)

In einem zweiphasigen System aus Wasser und einem organischen Lösungsmittel, vorzugsweise Methylenchlorid, bei -5°C bis 40°C, wobei der pH-Wert der wässrigen Phase durch Zugabe oder Vorlage eines Alkali- oder Erdalkalihydroxids zwischen 1 und 13, vorzugsweise zwischen 2 und 9 gehalten wird.

Dabei haben X, R¹, R², R³, R⁴ und m die in der Formel (I) genannte Bedeutung.

Bevorzugt sind an 1 - 2 Atomen X, insbesondere nur an einem Atom X pro Molekül (IV) R³ und R⁴ gleichzeitig Alkyl.

Bevorzugter Alkylrest ist Methyl; die X-Atome in α-Stellung zu dem Diphenylsubstituierten C-Atom (C-1) sind bevorzugt nicht dialkylsubstituiert, dagegen ist die Alkyldisubstitution in β-Stellung zu C-1 bevorzugt.

Bevorzugt sind Dihydroxydiphenylcycloalkane mit 5 und 6 Ring-C-Atomen im cycloaliphatischen Rest (m = 4 oder 5 in Formel (IV)), beispielsweise die Diphenole der Formeln (IVa) bis (IVc)) 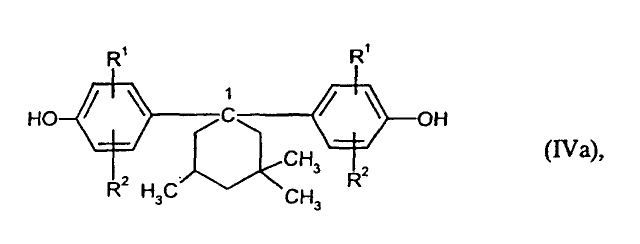 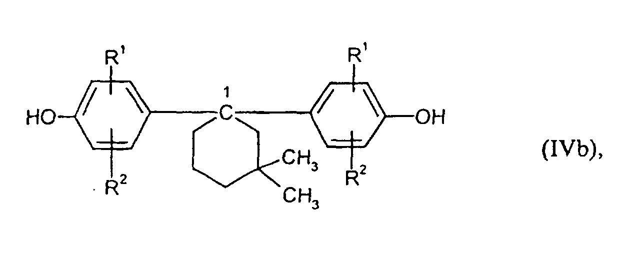 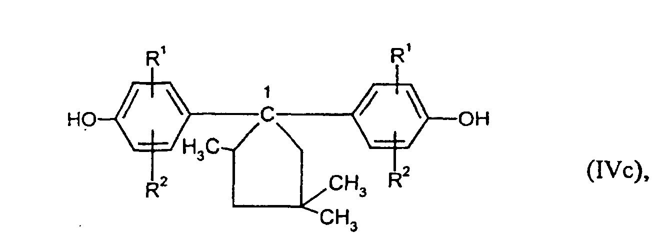 wobei das 1,1-Bis-(4-hydroxyphenyl)-3,3,5-trimethylcyclohexan (Formel (IVa) mit R¹ und R² gleich H) besonders bevorzugt ist. Die Polycarbonate können gemäß der deutschen Patentanmeldung P 3 832 396.6 aus Diphenolen der Formel (I) hergestellt werden.

Für die Wiederholeinheiten werden zusätzlich zu den Diphenolen der Formel (IV) andere Diphenole, beispielsweise mit der Formel (V)

HO-Z-OH (V)

verwendet.

Geeignete Diphenole der Formel (V) sind solche, in denen Z ein aromatischer Rest mit 6 bis 30 C-Atomen ist, der einen oder mehrere aromatische Kerne enthalten kann, substituiert sein kann und aliphatische Reste oder andere cycloaliphatische Reste als die der Formel (I) oder Heteroatome als Brückenglieder enthalten kann.

Beispiele der Diphenole der Formel (V) sind:
Hydrochinon, Resorcin, Dihydroxydiphenyle, Bis-(hydroxyphenyl)-alkane, Bis-(hydroxyphenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, α,α'-Bis-(hydroxyphenyl)-diisopropylbenzole sowie deren kemalkylierte und kernhalogenierte Verbindungen.

Bevorzugt sind α,α'-Bis-(hydroxyphenyl)-m-diisopropylbenzol, α,α'-Bis-(hydroxyphenyl)-p-diisopropylbenzol und 2,2-Bis-(4-hydroxyphenyl)-propan besonders bevorzugt α,α'-Bis-(hydroxyphenyl)-m-diisopropylbenzol und 2,2-Bis-(-hydroxyphenyl)-propan.

Diese und weitere geeignete Diphenole sind z.B. in der Monographie "H. Schnell, Chemistry and Physics of Polycarbonates, Interscience Publishers, New York 1964", beschrieben.

Natürlich können als Cobisphenole auch Mischungen von Bisphenolen (V) und weiteren Bisphenolen eingesetzt werden.

Dazu bevorzugte weitere Bisphenole sind beispielsweise:
4,4'-Dihydroxydiphenyl, 2,4-Bis-(4-hydroxyphenyl)-2-methylbutan, 1,1-Bis-(4-hydroxyphenyl)-cyclohexan, α,α'-Bis-(hydroxyphenyl)-p-diisopropylbenzol, 2,2-Bis-(3-methyl-4-hydroxyphenyl)-propan, 2,2-Bis-(3-chlor-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-4-hydroxyphenyl)-sulfon, 2,4-Bis-(3,4-dimethyl-4-hydroxyphenyl)-2-methylbutan, 1,1-Bis-(3,5-dimethyl-4-hydroxyphenyl)-cyclohexan, 2,2-Bis-(3,5-dichlor-4-hydroxyphenyl)-propan und 2,2-Bis-(3,5-dibrom-4-hydroxyphenyl)-propan.

Als Bisphenole der Formel (IV) werden vorzugsweise 1,1-Bishydroxyphenyl-3,3,5-trimethyl-cyclohexan oder 1,1-Bishydroxyphenyl-3-methyl-cyclohexan eingesetzt, besonders bevorzugt 1,1-Bishydroxyphenyl-3,3,5-trimethyl-cyclohexan.

Als Bisphenole der Formel (V) wurden vorzugsweise 2,2-Bishydroxyphenyl-propan (BPA), 1,1-Bishydroxyphenylethan oder 2,2-Bishydroxyphenylbutan, besonders bevorzugt 2,2-Bishydroxyphenylpropan eingesetzt.

Ein weiterer Gegenstand der Erfindung ist die Verwendung der erfindungsgemäßen Bischlorkohlensäureestergemische zur Herstellung cyclischer Cooligocarbonate.

Die Synthese der cyclischen Cooligocarbonate erfolgt durch synchrone Zugabe der Lösung der Bischlorkohlensäureestergemische in Methylenchlorid und 3 - 15 Mol-% eines organischen Amins, vorzugsweise Triethylamin, zu einer zweiphasigen Mischung aus Methylenchlorid und Wasser, wobei der pH-Wert der wässrigen Phase durch Zugabe oder Vorlage eine Alkali- oder Erdalkalihydroxids zwischen 7 und 13, vorzugsweise zwischen 9 und 11 gehalten wird und die Temperaur 0°C bis 40°C, vorzugsweise 30°C bis 40°C beträgt. Eventuell werden noch zusätzlich bis zu 10 Mol-% andere Bisphenole eingesetzt.

Ein weiterer Gegenstand der Erfindung ist die Verwendung der erfindungsgemäßen Bischlorkohlensäureestergemische zur Herstellung linearer statistischer Copolycarbonate.

Die hochmolekularen Polycarbonate aus den erfindungsgemäßen Bischlorkohlensäureestern, gegebenenfalls in Kombination mit anderen Bischlorkohlensäureestern, können nach den bekannten Polycarbonat-Herstellungsverfahren hergestellt werden, vorzugsweise nach dem Phasengrenzflächenverfahren (vgl. H. Schnell "Chemistry and Physics of Polycarbonates", Polymer Reviews, Vol. IX, Seite 33 ff., Interscience Publ. 1964).

Die Synthese der linearen, statistischen Copolycarbonate erfolgt z.B. durch eine Zweiphasenreaktion einer Lösung der Bischlorkohlensäureestergemische in Methylenchlorid mit in Wasser gelösten anorganischen Basen, vorzugsweise Alkalihydroxiden, bei einem pH-Wert von 7 - 13 und einer Temperatur von 0°C - 40°C in Gegenwart von 0,01 - 2 Mol-% eines organischen Amins, vorzugsweise Triethylamin oder N-Ethylpiperidin, wobei 0 - 80 Mol-% Bisphenole der Formel (IV) und (V) und 0 - 7 Mol-% Monophenole zugesetzt werden können.

Als Kettenabbrecher zur an sich bekannten Regelung des Molekulargewichts der Polycarbonate dienen monofunktionelle Verbindungen in üblichen Konzentrationen. Geeignete Verbindungen sind z.B. Phenol, tert.-Butylphenole oder andere Alkylsubstituierte Phenole. Zur Regelung des Molekulargewichts sind insbesondere kleine Mengen Phenole der Formel (VI) geeignet 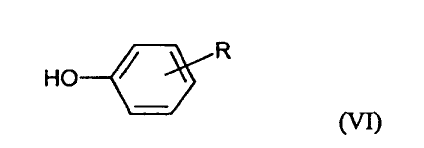 worin
- R: einen verzweigten C₈- und/oder C₉-Alkylrest darstellt.

Bevorzugt ist im Alkylrest R der Anteil an CH₃-Protonen zwischen 47 und 89 % und der Anteil der CH- und CH₂-Protonen zwischen 53 und 11 %; ebenfalls bevorzugt ist R in o- und/oder p-Stellung zur OH-Gruppe, und besonders bevorzugt die obere Grenze des ortho-Anteils 20 %. Die Kettenabbrecher werden im allgemeinen in Mengen von 0,5 bis 10, bevorzugt 1,5 bis 8 Mol-%, bezogen auf eingesetzte Diphenole, eingesetzt.

Die erhaltenen Copolycarbonate haben weder blockartige noch alternierende Strukturen. Sie sind statistisch aufgebaut.

Die Erfindung soll anhand der nachfolgenden Beispiele näher erläutert werden.

### Beispiele

### Beispiel 1

### Herstellung eines erfindungsgemäßen Bischlorkohlensäureestergemisches

In eine Mischung aus 155 g (0,5 mol) TMC-Bisphenol, 114 g (0,5 Mol) BPA und 1,21 Methylchlorid werden bei 0°C 300 g Phosgen in 150 Minuten eingeleitet. Gleichzeitig wird 25 proz. NaOH so zudosiert, dass der pH-Wert der Mischung zwischen 2 und 5 liegt. Man braucht dafür ca. 1 Liter NaOH. Nach der Phosgeneinleitung wird der pH-Wert auf 8 bis 9 eingestellt und solange Stickstoff eingeleitet, bis die Lösung phosgenfrei ist. Nach der Phasentrennung wird die organische Phase mit IN HCl und dann mit Wasser gewaschen, über Natriumsulfat getrocknet und eingedampft. Die Ausbeute betrug 285 g, der Gehalt an hydrolysierbarem Chlor 14,5 %. Ein Gemisch der nicht oligomerisierten Bischlorkohlensäureester würde theoretisch 18,0 % hydrolysierbares Chlor enthalten. Daher liegt ein Anteil von Oligochlorkohlensäureestern mit p, q, r und s > 1 vor.

### Beispiel 2

### Herstellung eines cyclischen Oligocarbonats

### (Verwendungsbeispiel)

In einem 1 Liter Kolben werden 200 ml Methylenchlorid, 7 ml Wasser, 2 ml 9,75 molare NaOH in Wasser und 2,4 ml Triethylamin vorgelegt und bei 40°C unter kräftigem Rühren synchron 200 ml einer Lösung von 78,8 g des Bischlorkohlensäureestergemisches aus Beispiel 1 in Methylenchlorid, 59 ml einer 0,75 molaren NaOH in Wasser und 25 ml einer 10 %igen Lösung von Triethylamin in Methylenchlorid innerhalb von 28 Minuten zudosiert, wobei die Dosierung der Bischlorkohlensäureesterlösung unterhalb des Flüssigkeitsspiegels erfolgt. Nach der Phasentrennung wird mit 1 N HCI und dreimal mit Wasser gewaschen und die organische Phase eingedampft. Man erhält 60,2 g eines Materials, das nach HPLC zu ca. 80 % aus Zyklen besteht.

### Beispiel 3

### Herstellung eines linearen Polycarbonats

### (Verwendungsbeispiel)

Man legt 39,4 g der Bischlorkohlensäureester nach Beispiel 1, 62 g 45 %ige NaOH, 0,53 g p-tert.-Butylphenol und 400 g Methylenchlorid vor, gibt 0,1 g N-Ethylpiperidin hinzu und läßt 1 h nachrühren. Man säuert mit HCl ab, trennt die organische Phase ab, und dampft sie im Vakuumtrockenschrank ein. Man erhält 30 g eines Polycarbonats mit einer relativen Lösungsviskosität von 1,29 (0,5 % in Methylenchlorid bei 25°C) mit einer Glastemperatur von 199°C.

## Patentansprüche

1. Gemische aus Bischlorkohlensäureestern der Formeln (I), (II) und (III) 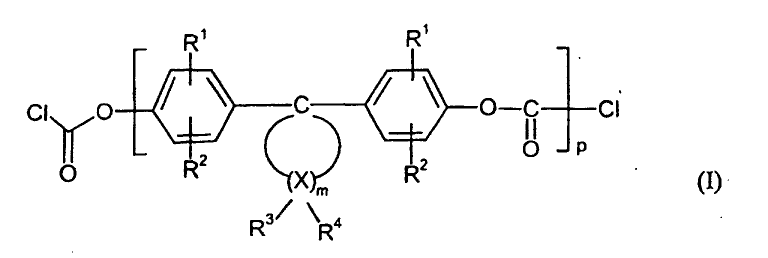 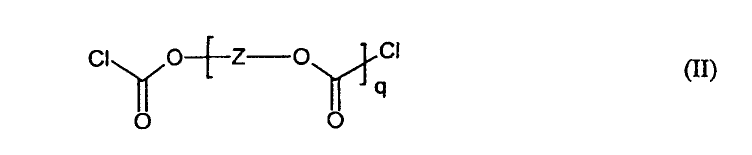 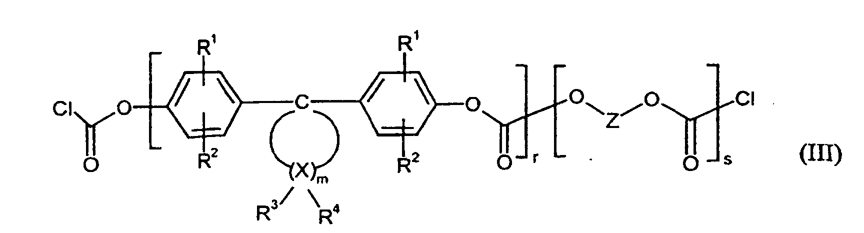 wobei
R¹ und R² unabhängig voneinander Wasserstoff, Halogen, bevorzugt Chlor oder Brom, C₁-C₈-Alkyl, C₅-C₆-Cycloalkyl, C₆-C₁₀-Aryl, bevorzugt Phenyl, und C₇-C₁₂-Aralkyl, bevorzugt Phenyl-C₁-C₄-Alkyl, insbesondere Benzyl,
m eine ganze Zahl von 4 bis 7, bevorzugt 4 oder 5,
R³ und R⁴ unabhängig voneinander Wasserstoffoder C₁-C₆-Alkyl und
X Kohlenstoff bedeuten,
mit der Maßgabe, dass an mindestens einem Atom X pro Wiederholungseinheit in (I) oder (III) R³ und R⁴ gleichzeitig Alkyl bedeuten,
Z ein aromatischer Rest mit 6 bis 30 C-Atomen ist, der einen oder mehrere aromatische Kerne enthält, substituiert sein kann und aliphatische Reste oder cycloaliphatische Reste die unterschiedlich zu den cycloaliphatischen Resten in Formel (I) sind, oder Heteroatome als Brückenglieder enthalten kann,
p, q, r und s für eine natürliche Zahl von 1 bis 15 stehen und das molare Verhältnis der cycloaliphatischen Bisphenoleinheiten zu den aliphatischen Bisphenoleinheiten 1:10 bis 10:1 beträgt.

2. Gemische gemäß Anspruch 1, wobei
Z den Resten 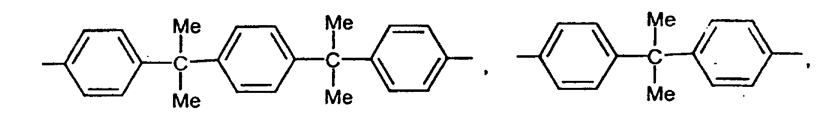 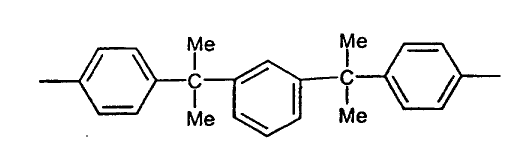 entspricht,
wobei Me Methylrest bedeutet.

3. Verwendung der Bischlorkohlensäureestergemische nach Anspruch 1 oder 2 zur Herstellung cyclischer Cooligocarbonate.

4. Verwendung der Bischlorkohlensäureestergemische nach Anspruch 1 oder 2 zur Herstellung linearer statistischer Copolycarbonate.

## Claims

1. Mixtures of bischloroformic acid esters corresponding to formulae (I), (II) and (III) 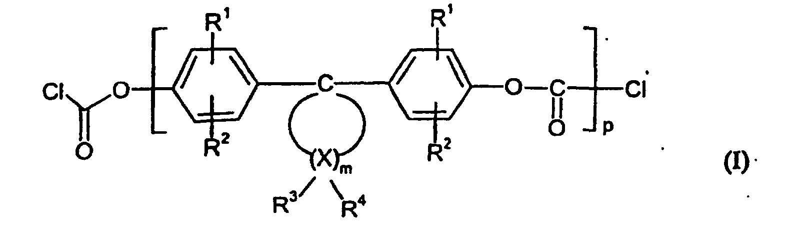 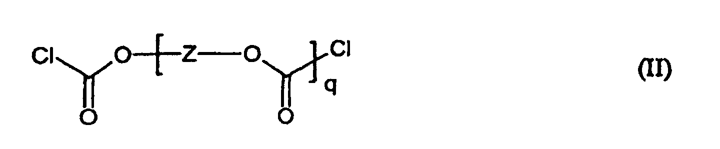 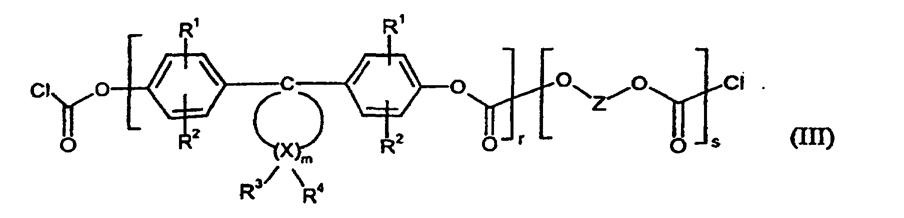 wherein
R¹ and R² independently of one another denote hydrogen, halogen, preferably chlorine or bromine, C₁-C₈-alkyl, C₅-C₆-cycloalkyl, C₆-C₁₀-aryl, preferably phenyl, and C₇-C₁₂-aralkyl, preferably phenyl-C₁-C₄-alkyl, in particular benzyl,
m is an integer from 4 to 7, preferably 4 or 5,
R³ and R⁴ independently of one another denote hydrogen or C₁-C₆-alkyl and
X denotes carbon,
with the proviso that, on at least one X atom per repeat unit in (I) or (III), R³ and R⁴ simultaneously denote alkyl,
Z is an aromatic group having 6 to 30 C atoms and can contain one or more aromatic nuclei, can be substituted and can contain aliphatic groups or cycloaliphatic groups which differ from the cycloaliphatic groups in formula (I), or hetero atoms as bridge-type cross-links,
p, q, r and s represent a natural number from 1 to 15 and the molar ratio of the cycloaliphatic bisphenol units to the aliphatic bisphenol units is 1:10 to 10:1.

2. Mixtures according to claim 1, wherein
Z corresponds to the groups 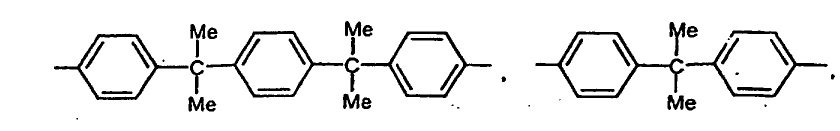 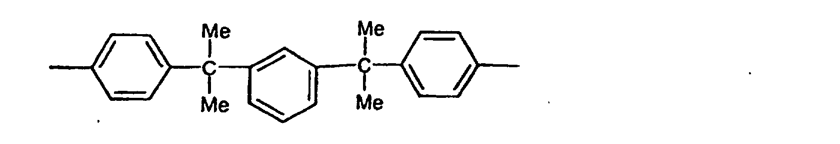 wherein Me denotes a methyl group.

3. Use of the mixtures of bischloroformic acid esters according to claim 1 or 2 for the production of cyclic cooligocarbonates.

4. Use of the mixtures of bischloroformic acid esters according to claim 1 or 2 for the production of linear statistical copolycarbonates.

## Revendications

1. Mélange d'esters d'acide bischlorocarbonique répondant aux formules (I), (II) et (III) 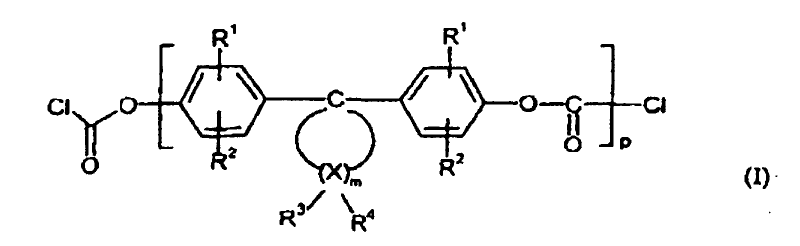 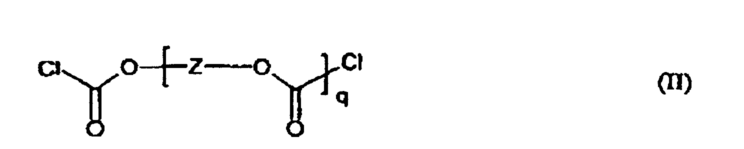 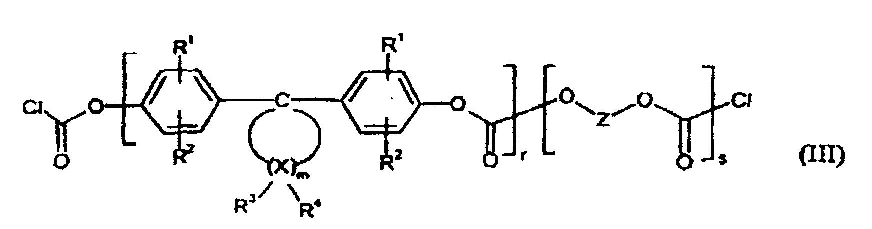 dans lesquelles:
R¹ et R² représentent, indépendamment l'un de l'autre, un atome d'hydrogène, un atome d'halogène, de préférence chlore ou brome, un groupe alkyle en C₁-C₈, cycloalkyle en C₅-C₆, aryle en C₆-C₁₀, de préférence phényle, et aralkyle en C₇-C₁₂, de préférence phényl (alkyle en C₁-C₄), en particulier benzyle,
m représente un nombre entier de 4 à 7, de préférence 4 ou 5,
R³ et R⁴ sont, indépendamment l'un de l'autre, un atome d'hydrogène ou un groupe alkyle en C₁-C₆, et
X représente un atome de carbone,
avec, comme précision que sur au moins un atome X par unité répétitive dans (I) ou (III), R³ et R⁴ représentent en même temps alkyle,
Z représente un reste aromatique comportant 6 à 30 atomes de C, qui contient un ou plusieurs noyaux aromatiques, qui peut être substitué et peut contenir, comme membres de pontage, des restes aliphatiques ou des restes cycloaliphatiques qui sont différents des restes cycloaliphatiques présents dans la formule (I) ou des hétéroatomes,
p, q, r et s représentent un nombre naturel de 1 à 15, et le rapport molaire des unités de bisphénol cycloaliphatiques aux unités de bisphénol aliphatiques est de 1:10 à 10:1.

2. Mélange selon la revendication 1, dans lequel
Z correspond aux restes 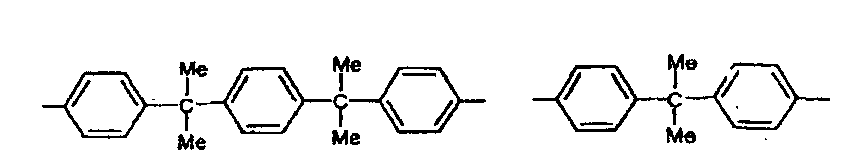 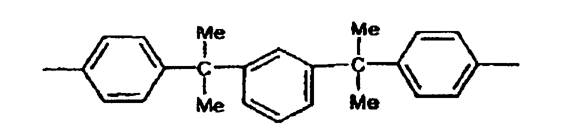 Me signifiant reste méthyle.

3. Utilisation de mélanges d'esters d'acide bischlorocarbonique selon la revendication 1 ou 2, pour la préparation de cooligocarbonates cycliques.

4. Utilisation de mélanges d'esters d'acide bischlorocarbonique selon la revendication 1 ou 2 pour la préparation de copolycarbonates statistiques linéaires.
